(19) Europäisches Patentamt
European Patent Office
Office européen des brevets

(11) Publication number: 0 533 926 A1

# (12) EUROPEAN PATENT APPLICATION published in accordance with Art. 158(3) EPC

(21) Application number: 91911183.1

(22) Date of filing: 13.06.91

(86) International application number: PCT/JP91/00795

(87) International publication number: WO 91/19728 (26.12.91 91/29)

(51) Int. Cl.⁵: C07H 19/213

(30) Priority: 15.06.90 JP 158233/90

(43) Date of publication of application: 31.03.93 Bulletin 93/13

(84) Designated Contracting States: AT BE CH DE DK ES FR GB GR IT LI LU NL SE

(71) Applicant: NIPPON SHINYAKU COMPANY, LIMITED
14, Kisshoin Nishinosho Monguchicho
Minami-ku Kyoto-shi Kyoto 601(JP)

(72) Inventor: YANO, Junichi
840-86, Sahodai 2-chome Nara-shi
Nara 630(JP)
Inventor: OHGI, Taadaaki
24-33, Kokubu 2-chome
Otsu-shi Shiga 520(JP)
Inventor: ISHIYAMA, Kouichi
51-22 Z-405, Aza Enmyoji Koaza Kuzuhara
Oyamazakicho Otokuni-gun Kyoto 618(JP)

(74) Representative: Alber, Norbert et al
Patent- und Rechtsanwälte-Hansmann,
Vogeser, Boecker, Alber,
Albert-Rosshaupter-Strasse 65
W-8000 München 70 (DE)

## (54) NUCLEOTIDE DERIVATIVE.

(57) A nucleotide derivative represented by general formula (I), which has an excellent pharmacological action and is obtained from a structural derivative other than naturally occurring normal cyclic nucleotides: wherein $R^1$ represents hydrogen, hydroxy, acyloxy or alkoxy; $R^2$ represents alkyl; and $R^3$ represents hydrogen, halogen, hydroxy or alkyl.

EP 0 533 926 A1

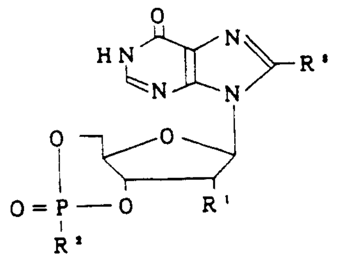

[I]

TECHNICAL FIELD

The present invention relates to cyclic IMP derivatives which are suitable for the treatment of angina pectoris and hypertension, among other things.

The compounds according to the invention are novel compounds which may be represented by the following general formula [I] and include optically pure isomers.

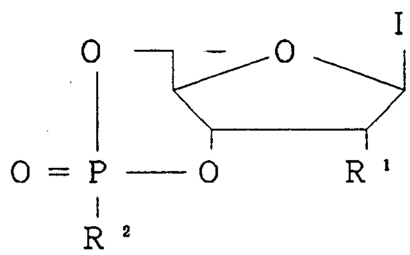

[ I ]

wherein I represents

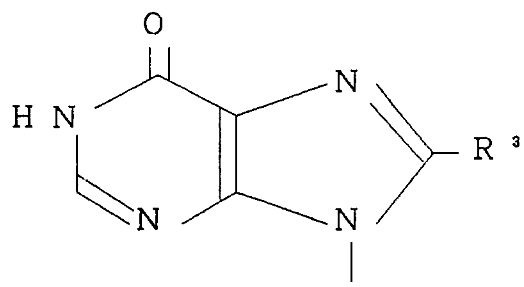

; $R^1$ represents hydrogen, hydroxy, acyloxy or alkoxy; $R^2$ represents alkyl;

$R^3$ represents hydrogen, halogen, hydroxy or alkyl.

BACKGROUND ART

It is known that nucleotide derivatives such as cyclic AMP are physiological mediators for hormones, for instance, and are associated with various physiological interactions in the living body, thus gathering a major attention to their pharmacologic effects.

Nucleotide derivatives such as cyclic AMP are, however, dubious in the aspect of utility as a medicament because they are poorly absorbed and if absorbed are liable to be decomposed by endogenous enzymes.

The inventors of thou present invention found after much research on nucleotide derivatives that those non-dissociated cyclic AMP derivatives and cyclic GMP derivatives which are obtainable by modifying the phosphoric acid moiety of the nucleotide molecule have desirable pharmacologic activities and disclosed the findings in an application for patent (Japanese Patent Application No. 1-207192/1989).

Cyclic AMP and cyclic GMP are distributed broadly in the natural kingdom and their functions have already been elucidated. However, it is not known that cyclic IMP is functioning as a second messenger in the living body. Therefore, the inventors made further explorations and found that non-dissociated cyclic IMP derivatives constitute a group of compounds having pharmacologic actions by far suspassing those of the analogs thus far known. The present invention has been developed on the basis of the above findings.

DISCLOSURE OF INVENTION

It was, therefore, an object of the invention to find out nucleotide derivatives having superior pharmacologic activities from among structural derivatives other than the ordinary naturally-occurring cyclic nucleotides.

The technical problem to which the inventors addressed themselves was derivation of pharmaceutical efficacies far exceeding the efficacies of the hithereto-known nucleotide derivatives.

The compound of the present invention can be represented by the general formula [I] presented above. This very structure constitutes the substantive element of the present invention.

The present invention was implemented only through the discovery that said compound has the pharmacologic activities described hereinafter.

Of course, optically pure compounds are also subsumed in the concept of compound of the invention.

Referring, now, to general formula [I], I has the skeletal structure of inosine.

Here, $R^1$ represents hydrogen, hydroxy, acyloxy or alkoxy. The acyloxy includes, among others, acetyloxy, n-propionyloxy, isopropionyloxy, n-butyryloxy, iso-butyryloxy, sec-butyryloxy, tert-butyryloxy, etc. and the alkoxy includes, among others, methoxy, ethoxy, n-propoxy, isopropoxy, sec-butoxy, tert-butoxy, tetra-hydropyranyloxy and so on. $R^2$ represents an alkyl group of the common sort, inclusive of lower alkyls, such as methyl, ethyl, propyl, isopropyl, n-butyl, isobutyl, sec-butyl, tert-butyl and so on. $R^3$ represents hydrogen, halogen, hydroxy or alkyl. The halogen is chlorine, fluorine, bromine or iodine, for instance, and the alkyl includes, among others, methyl, ethyl, propyl, isopropyl, n-butyl, isobutyl, sec-butyl, tert-butyl and so on.

The compound of the present invention subsumes the following compounds but is not limited thereto. Of course, each of these compounds of the invention includes an optically pure isomer and its diastereomer.

Inosine-3',5'-cyclic methylphosphonate, inosine-3',5'-cyclic ethylphosphonate, inosine-3',5'-cyclic propylphosphonate, 2'-deoxyinosine-3',5'-cyclic methylphosphonate, 2'-deoxyinosine-3',5'-cyclic ethylphosphonate, 2'-deoxyinosine-3',5'-cyclic propylphosphonate, 2'-O-butyrylinosine-3',5'-cyclic methylphosphonate, 2'-O-butyrylinosine-3',5'-cyclic ethylphosphonate, 2'-O-butyrylinosine-3',5'-cyclic propylphosphonate, 8-chloroinosine-3',5'-cyclic methylphosphonate, 8-chloroinosine-3',5'-cyclic ethylphosphonate, 8-chloro-inosine-3',5'-cyclic propylphosphonate, 8-chloro-2'-deoxyinosine-3',5'-cyclic methylphosphonate, 8-chloro-2'-deoxyinosine-3'5'-cyclic ethylphosphonate, 8-chloro-2'-deoxyinosine-3',5'-cyclic propylphosphonate, 8-chloro-2'-O-butyrylinosine-3',5'-cyclic methylphosphonate, 8-chloro-2'-O-butyrylinosine-3',5'-cyclic ethylphosphonate, 8-chloro-2'-O-butyrylinosine-3',5'-cyclic propylphosphonate, 8-bromoinosine-3',5'-cyclic methylphosphonate, 8-bromoinosine-3',5'-cyclic ethylphosphonate, 8-bromoinosine-3',5'-cyclic propylphosphonate, 8-bromo-2'-deoxyinosine-3',5'-cyclic methylphosphonate, 8-bromo-2'-deoxyinosine-3',5'-cyclic ethylphosphonate, 8-bromo-2'-deoxyinosine-3',5'-cyclic propylphosphonate, 8-bromo-2'-O-butyrylinosine-3',5'-cyclic methylphosphonate, 8-bromo-2'-O-butyrylinosine-3',5'-cyclic ethylphosphonate, 8-bromo-2'-O-butyrylinosine-3',5'-cyclic propylphosphonate, 8-methylinosine-3',5'-cyclic methylphosphonate, 8-methylinosine-3',5'-cyclic ethylphosphonate, 8-methylinosine-3',5'-cyclic propylphosphonate, 8-methyl-2'-deoxyinosine-3',5'-cyclic methylphosphonate, 8-methyl-2'-deoxyinosine-3',5'-cyclic ethylphosphonate, 8-methyl-2'-deoxyinosine-3',5'-cyclic propylphosphonate, 8-methyl-2'-O-butyrylinosine-3',5'-cyclic methylphosphonate, 8-methyl-2'-O-butyrylinosine-3'5'-cyclic ethylphosphonate, 8-methyl-2'-O-butyrylinosine-3',5'-cyclic propylphosphonate, 8-isopropylinosine-3',5'-cyclic methylphosphonate, 8-isopropylinosine-3',5'-cyclic ethylphosphonate, 8-isopropylinosine-3',5'-cyclic propylphosphonate, 8-isopropyl-2'-deoxyinosine-3',5'-cyclic methylphosphonate, 8-isopropyl-2'-deoxyinosine-3',5'-cyclic ethylphosphonate, 8-isopropyl-2'-deoxyinosine-3',5'-cyclic propylphosphonate, 8-isopropyl-2'-O-butyrylinosine-3',5'-cyclic methylphosphonate, 8-isopropyl-2'-O-butyrylinosine-3',5'-cyclic ethylphosphonate, 8-isopropyl-2'-O-butyrylinosine-3',5'-cyclic propylphosphonate, 8-hydroxyinosine-3',5'-cyclic methylphosphonate, 8-hydroxyinosine-3',5'-cyclic ethylphosphonate, 8-hydroxyinosine-3',5'-cyclic propylphosphonate, 8-hydroxy-2'-deoxyinosine-3',5'-cyclic methylphosphonate, 8-hydroxy-2'-deoxyinosine-3',5'-cyclic ethylphosphonate, 8-hydroxy-2'-deoxyinosine-3',5'-cyclic propylphonsphonate, 8-hydroxy-2'-O-butyrylinosine-3',5'-cyclic methylphosphonate, 8-hydroxy-2'-O-butyrylinosine-3',5'-cyclic ethylphosphonate, and 8-hydroxy-2'-O-butyrylinosine-3',5'-cyclic propylphosphate.

Experimental Example

Inhibitory effect on human platelet aggregation

Using a syringe filled with 2.5 ml of sodium citrate solution (Citrate for erythrocyte sedimentation test (Kokusai) distributed by Green Cross Corporation) beforehand, human blood was collected in a total quantity of 25 ml and stirred well. The blood was then centrifuged at 200× g for 10 minutes and the supernatant was recovered as a PRP (platelet-rich plasma).

Horse tendon collagen [HORMON-CHEMIE MÜNCHEN GMBH, collagen reagent (Holm)] was previously diluted to 10 and 20 $\mu$g /ml with the accompanying buffer.

Each test drug was diluted with PBS and its concentration (mM) was estimated from ultraviolet absorption.

The assay for platelet aggregation was carried out with NKK HEMA TRACER 1 (Nikko Bioscience). The cell was filled with 0.2 ml of PRP and 0.025 ml of the test drug solution and the absorbance was measured at a constant temperature of 37 °C. Then, 0.025 ml of the above collagen solution was added and the absorbance was measured (wavelength 660 nm). The percent inhibition of platelet aggregation (%) was calculated by means of the following equation.

$$\text{Percent inhibition of platelet aggregation (\%)} = 100 - \frac{[\text{PRP + test drug + collagen}] - [\text{PRP + test drug}]}{[\text{PRP + PBS + collagen}] - [\text{PRP + PBS}]} \times 100$$

[ ] represents the absorbance.

The results are shown below.

| Test drug | 10 μM * | 100 μM * |
|---|---|---|
| c-GMP | 4.0% | 23% |
| 8-Br-c-GMP | 2.9% | 49% |
| c-IMP methylphosphonate | 56 % | 86% |
| 8-Br-c-IMP methylphosphonate | 57 % | - |
| The concentration of collagen is 2 μg/ml. | | |

* The concentrations of the test drugs were calculated using the following ε values.
c-GMP (manufactured by Sigma, λ max 253 nm (ε :14,000))
8-Br-c-GMP (manufactured by Sigma, λ max 263 nm (ε :15,300))
c-IMP methylphosphonate (manufactured by Sigma, λ max 248.5 nm (ε :11,600))
8-Br-c-IMP methylphosphonate (manufactured by Sigma, λ max 253.5 nm (ε :14,400))

It is clear from the above data that the compounds of the invention have platelet aggregation inhibitory activity. Thus, compared with cyclic GMP and 8-bromocyclic GMP, the compounds of the invention exhibited 10-fold as high activity at about 50% inhibitory concentration.

For administration as a drug to animals including man, the compound of the invention can be used either as it is or as a pharmaceutical composition prepared to contain the compound in a pharmaceutically acceptable nontoxic and inert excipient at a level of, for example, 0.1% to 99.5%, preferably 0.5% to 90%.

As the excipient, one or more solid, semisolid or liquid diluents, fillers and other auxiliary excipients can be employed. The pharmaceutical composition is preferably administered in unit dosage forms. The pharmaceutical composition of the present invention can be administered orally, into the tissue, locally (e.g. transdermally) or rectally. Of course, dosage forms should be tailored to the respective routes of administration. Administration into the tissue, for instance, is particularly preferred.

The dosage for use as a platelet aggregation inhibitor is preferably adjusted according to the patient's characteristics such as age and body weight, the route of administration, the type and severity of disease and so on. Generally, however, the effective dose for an adult human is 100 mg - 3 g/day/body and preferably 500 mg - 1 g/day/body. There are cases in which a dosage either less or more than the above range is sufficient. The daily dosage is preferably administered in 1 to 3 divided doses.

The cyclic nucleotide derivatives according to the present invention can be synthesized, for example by the reactions schematically shown below.

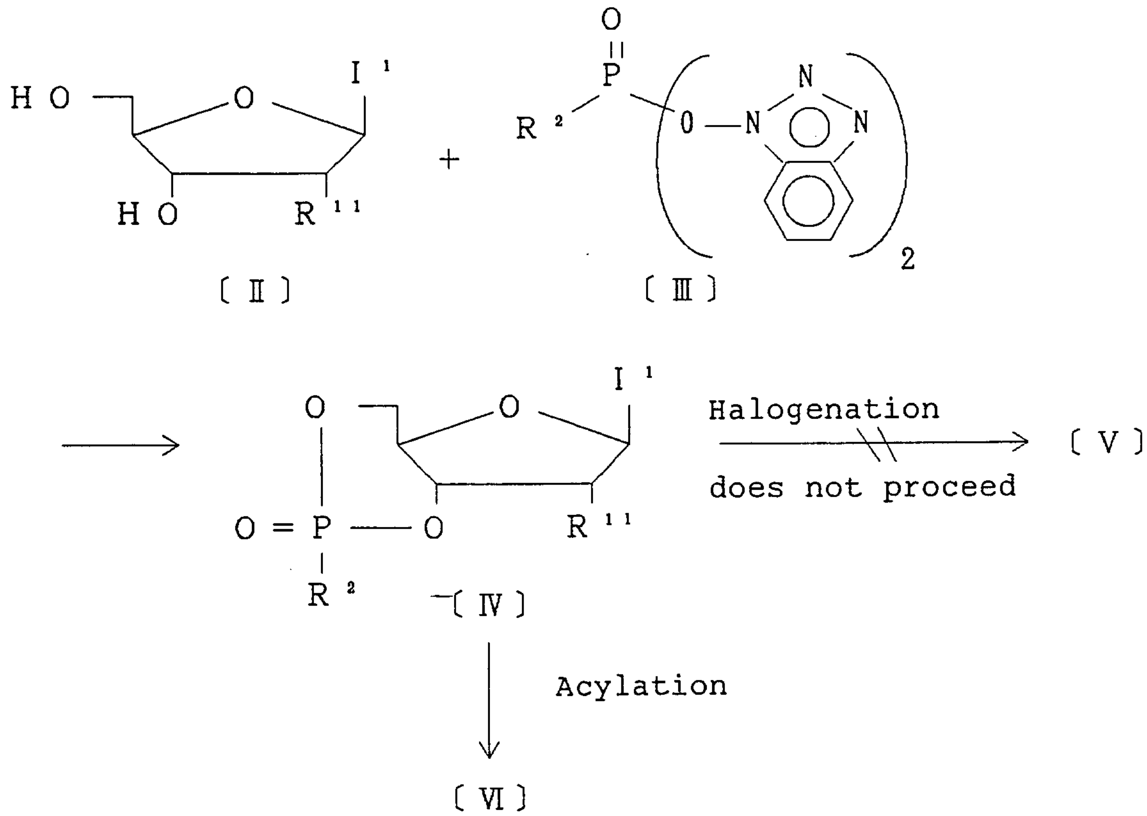

[II] [III] [IV] [V] [VI]

(wherein $R^2$ is as defined hereinbefore; $I^1$ represents hypoxanthine; $R^{11}$ represents hydrogen or tetrahydropyranyloxy.)

Thus, a nucleoside of general formula [II] is reacted with an alkyl O,O-bis(1-benzotriazolyl)phosphonate of general formula [III] and, then, an 1-alkylimidazole, such as 1-methylimidazole, is added to give the cyclic derivative [IV]. If necessary, the tetrahydropyranyl group is eliminated by reaction with an acid.

The compound of general formula [IV] can then be acylated to a compound of general formula [VI] (2'-acyloxy derivative).

The starting compound [II] for use in this process can be prepared by the method described in the literature [S. Honda et al., Tetrahedron, 40, 153-163,(1984)] or any process analogous thereto. The compound of general formula [III] can be prepared from an alkylphosphonic acid dichloride and 1-hydroxybenzotriazole by the method of van Boon et al. [J.H. van Boom et al., Nucleic Acids Research 14, 2171-2185 (1986)].

The reaction of [II] with [III] is conducted in a solvent inert to the reaction (for example, ethers such as anhydrous dioxane and tetrahydrofuran), generally by allowing the reaction mixture to stand at room temperature for 30 minutes to 3 hours and, after addition of an 1-alkylimidazole, further at room temperature for 5 to 24 hours.

The proportion of [III] is preferably 1 to 1.2 moles per mole of [II]. The proportion of said 1-alkylimidazole is preferably 3 to 7 moles per mole of the product of reaction of [II] with [III].

Then, the reaction mixture is allowed to stand in the presence of a strong acid at pH about 2.0, preferably a volatile acid such as trifluoroacetic acid or hydrochloric acid, at 0°C - room temperature for 5 - 24 hours, whereby [IV] is obtained. Generally, the acid is preferably used in a large excess over [IV].

[IV] cannot be converted to [V] by the ordinary bromination procedure, such as the reaction with an aqueous solution of bromine in acetate buffer or with an aqueous solution of bromine. Therefore, [V] is synthesized by an alternative process to be described hereinafter.

[IV] can be acylated to [VI] using an acid halide or anhydride corresponding to the acyl $R^3$. This acylation reaction can be carried out in pyridine at 0°C - room temperature for a few to 24 hours.

Moreover, [IV] and [V] can be synthesized by the reactions schematically shown below.

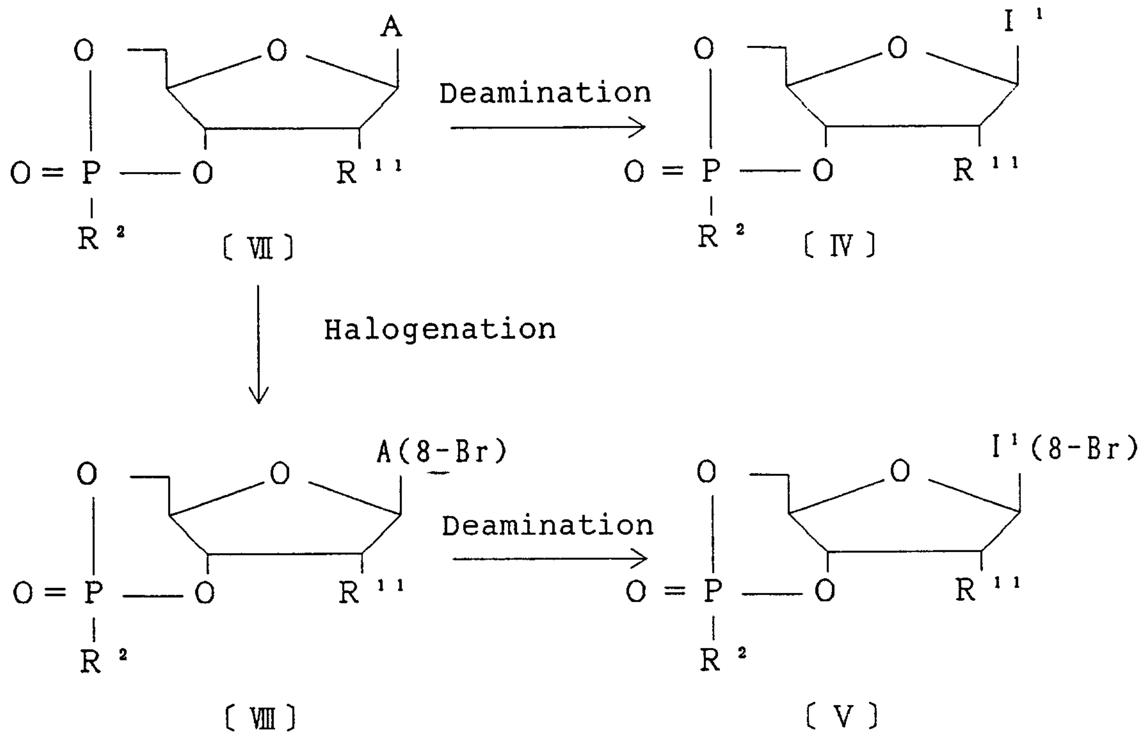


[VII] [IV] [VIII] [V]

(wherein $R^2$ is as defined hereinbefore. $I^1$ stands for hypoxanthine, A for adenine, A (8-Br) for 8-bromoadenine, and $I^1$ (8-Br) for 8-bromohypoxanthine. $R^{11}$ represents hydrogen, hydroxy, acyloxy or alkoxy)

[VII] and [VIII] can be synthesized by known processes (Japanese Application No. 1-207192/1989). Deamination of [VII] with sodium nitrite in acetic acid at room temperature gives [IV] in quantitative yield. Moreover, [VIII] can be similarly deaminated to give [V].

Furthermore, the compound of the invention which is available on substitution of the 8-position of hypoxanthine with a hydroxyl group can be synthesized by halogenating the 8-position of hypoxanthine in general formula [II] by the conventional procedure in the first place, substituting the halogen with a hydroxyl group by a method analogous to the one described in the literature [M. Ikehara et al., Tetrahedron, 24, 3489 (1968)], and cyclizing the resulting compound by the same procedure as above.

The compound of the invention which is available on substitution of the 8-position of hypoxanthine with an alkyl group can be synthesized by direct alkylation in accordance with the method described in the literature [M. Maeda et al., Tetrahedron, 30, 2677 (1974)] and subsequent cyclization by the same procedure as above.

Each of the object compounds which are thus produced can be isolated and purified by per se known procedures such as solvent extraction, pH adjustment, redistribution, concentration, crystallization, recrystallization and chromatography.

## BEST MODE FOR CARRYING OUT THE INVENTION

The following production examples for the compound of the invention are further illustrative of the present invention.

The following substance used in the examples was prepared as follows.

N-Monomethoxytrityl-2'-O-tetrahydropyranylinosine was prepared in accordance with the process described in the literature [S. Honda et al., Tetrahedron, 40, 153-163 (1984)].

Example 1 Synthesis of inosine-3',5'-cyclic methylphosphonate

To 113 mg of N-monomethoxytrityl-2'-O-tetrahydropyranylinosine [II] was added 3.90 ml of a 0.11 M solution of methyl-O,O-bis(1-benzotriazolyl)phosphonate in dioxane as prepared by the method of van Boom et al. [J.H. van Boom et al., Nucleic Acids Research, 14, 2171-2185 (1986)] at room temperature and

30 minutes later 0.16 ml of 1-methylimidazole was added. The reaction was carried out overnight and after 0.5 ml of 50% aqueous pyridine was added, the reaction mixture was concentrated to about half the volume. The concentrate was distributed between dichloromethane and water (a saturated aqueous solution of sodium chloride; 2/10 by volume) and the organic layer was dried over magnesium sulfate and concentrated to dryness to recover a crude solid. This solid was purified by preparative TLC (6% methanol/dichloromethane) to give 30 mg of compound [IV] ($I^1$ = hypoxanthine, $R^2$ = methyl, $R^{11}$ = tetrahydropyranyl) as a white solid.

This product was dissolved in 2 ml of dioxane/water (9/1) and the solution was adjusted to pH 2.0 with 0.1N hydrochloric acid added dropwise and, then, allowed to stand at room temperature overnight. After confirmation of the completion of reaction by TLC, the reaction mixture was concentrated to dryness. The residue was purified by reversed phase chromatography in the same manner as the alternative process using sodium nitrite (described elsewhere) to give 10 mg of compound [IV] ($I^1$ = hypoxanthine, $R^2$ = methyl, $R^{11}$ = hydroxy) as a white solid.

Example 2 Synthesis of inosine-3'.5'-cyclic methylphosphonate (alternative process)

In 3.0 ml of 2N acetic acid was dissolved 30 mg of adenosine-3,5'-cyclic methylphosphonate [VII] ($R^2$ = methyl, $R^{11}$ = hydroxy) followed by addition of 1.8 ml of an aqueous solution of sodium nitrite (1 g of sodium nitrite in 3.75 ml of water), and the mixture was allowed to stand at room temperature for 2 hours and further at 4 ° C overnight. After completion of the reaction, the reaction mixture was concentrated to dryness by means of an aspirator and a vacuum pump and the residue was diluted with water (30 ml) and purified by reversed phase open chromatogrpahy.

Column: Capcell pak $C_{18}$ AG120 S-20/40 µm($\phi$ 1.7 × 7.5 cm)

Eluent: A linear gradient of water (150 ml) → 8% methanol/water (150 ml).

The main fractions were pooled, concentrated to dryness and pulverized from acetone - ethanol to give 24.0 mg of the object compound [IV] ($R^2$ = methyl, $R^{11}$ = hydroxy) as a white solid.

Melting point: 209-211 ° C (decomp.)

UV ($H_2O$): λ max 248.5 nm (ε : 11,600, neutral)

HPLC: 11.95 (min) (single peak); Capcell pak SG120 (S-5) $\phi$ 4.6× 150 mm, 260 nm, 1 ml/min, 22 ° C, 10% methanol/50 mM $KHPO_4$ (pH 7.0) 2mM TBHS

FAB MS: 329 ($MH^+$)

Example 3 Synthesis of 8-bromoinosine-3',5'-cyclic methylphosphonate (Sp-configuration )

To a solution of 17.8 mg of 8-bromoadenosine-3',5'-cyclic phosphonate [VIII] ($R^2$ = methyl, $R^{11}$ = hydroxy) in 2.0 ml of 2N acetic acid was added 1.2 ml of aqueous sodium nitrite solution and the mixture was allowed to stand at room temperature for 2.5 hours and further at 4 ° C overnight. After confirmation of the complete disappearance of the starting compound by micro-TLC (eluent: 10% methanol/dimethylmethane; starting compound Rf = 0.45, product compound Rf = 0.20), the reaction mixture was concentrated to dryness by means of an aspirator and a vacuum pump. The residue was dissolved in water (30 ml) and purified by reversed phase open chromatograhy.

Column: Capcell pak $C_{18}$ AG120 S-20/40 µm ($\phi$ 1.7× 7.5 cm)

Eluent: A linear gradient of water (150 ml) → 20% methanol/water (150 ml).

The main fractions were pooled and concentrated to dryness to give 19.1 mg of the expected compound [V] ($R^2$ = methyl, $R^{11}$ = hydroxy; Sp-configuration) as a white powder. This powder was recrystallized from n-hexane-dichloroethane-methanol.

Melting point 231-233 ° C (decomp.)

UV (H2O): λ max 253.5 nm (ε :14,400, neutral)

HPLC : 10.70 (min); Capcell pak SG120 (S-5), $\phi$ 4.6× 150 mm, 260 nm, 1 ml/min, 22 ° C , 10% $CH_3CN$/50 mM TEAA (pH 7.0) TEAA: Triethylammonium acetate

FAB MS : 407 ($MH^+$), 409 ($M+2H^+$)

1H-NMR ($D_2O$, 220 MHz):δ

1.84 (3H, d, J = 18.0 Hz, P-$CH_3$), 4.30-4.80 (3H, m, H-4', H-5'), 5.10 (1H, d, J = 6.0, H-2'), 5.50 (1H, m, H = 3'), 6.18 (1H, s, H-1'), 8.17 (1H, s, H-2)

| Elemental analysis (for $C_{11}H_{12}O_6N_4PBr \cdot H_2O$) | | | |
|---|---|---|---|
| | C | H | N |
| Calcd. (%) | 31.08 | 3.32 | 13.18 |
| Found (%) | 31.65 | 3.20 | 13.05 |

The diastereomer (Rp-configuration) of this expected compound [V] was eluted prior to said main fractions in the reversed phase open chromatography (yield 1.0 mg).

UV ($H_2O$): $\lambda$ max 253.5 nm (neutral)

HPLC: 8.06 (min); Capcell pak SG120 (S-5), $\phi$ 4.6$\times$ 150 mm, 260 nm, 1 ml/min, 22°C, 10% $CH_3CN$/50 mM TEAA (pH 7.0)

TEAA: Triethylammonium acetate

FAB MS: 407 ($MH^+$), 409 ($M + 2H^+$)

$^1$H-NMR ($D_2O$, 220 MHz):$\delta$

1.92 (3H, d, J=18.0 Hz, P-$CH_3$), 4.40-5.10 (4H, m), 5.32 (1H, m, H-3'), 6.20 (1H, s, H-1'), 8.16 (1H, s, H-2)

## Claims

**1.** A compound of the following general formula [I]

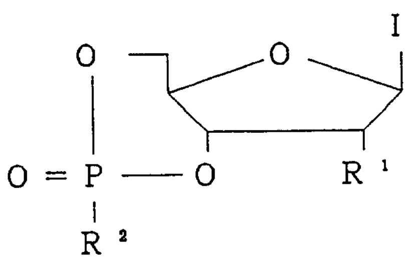

[ I ]

(wherein I represents

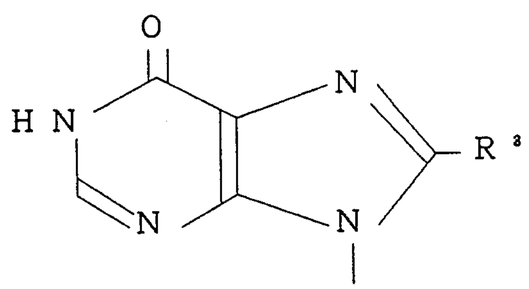

;

$R^1$ represents hydrogen, hydroxy, acyloxy or alkoxy; $R^2$ represents alkyl; and $R^3$ represents hydrogen, halogen, hydroxy or alkyl.

**2.** A compound according to claim 1, which compound is optically pure.

# INTERNATIONAL SEARCH REPORT

International Application No PCT/JP91/00795

**I. CLASSIFICATION OF SUBJECT MATTER** (if several classification symbols apply, indicate all) [6]

According to International Patent Classification (IPC) or to both National Classification and IPC

Int. $Cl^5$ C07H19/213//A61K31/70

**II. FIELDS SEARCHED**

Minimum Documentation Searched [7]

| Classification System | Classification Symbols |
|---|---|
| IPC | C07H19/213, 19/11 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 63-135399 (Akira Kaji), June 7, 1988 (07. 06. 88), (Family: none) | 1-2 |
| A | EP, A2, 355899 (NIPPON SHINYAKU KK), February 28, 1990 (28. 02. 90), & GB, A, 2222164 & FR, A, 2635526 & JP, A, 02-223590 | 1-2 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| August 20, 1991 (20. 08. 91) | September 2, 1991 (02. 09. 91) |
| **International Searching Authority** | **Signature of Authorized Officer** |
| Japanese Patent Office | |